# EUROPEAN PATENT APPLICATION

(11) **EP 2 550 956 A1**
(43) Date of publication of application: **30.01.2013**
(21) Application number: 11175298.6
(22) Date of filing: 26.07.2011
(51) Int. Cl.: A61K 8/891, A61K 8/34, A61K 8/31, A61Q 5/12

(54) **Non-aqueous composition for hair**

(71) Applicant: KPSS-Kao Professional Salon Services GmbH, 64297 Darmstadt (DE)
(72) Inventor: Herzog-Renker, Beate, 64291 Darmstadt (DE); Lipinski, Normen, 60325 Frankfurt (DE)

(57) **Abstract**

Present invention relates to a substantially non-aqueous composition for hair for conditioning hair. Accordingly, present invention is a substantially non-aqueous composition for hair comprising one or more alkyl methicone, one or more volatile organic solvent and one or more volatile isoalkane with the condition that the composition comprises not more than 5% by weight, water, calculated to the total composition.

## Description

Present invention relates to a substantially non-aqueous composition for conditioning hair.

Various types of compositions, mainly aqueous compositions, are used for hair conditioning. Hair conditioning is usually carried out after cleansing hair and the conventional products are applied onto wet hair and usually rinsed off after certain period of processing time. In the recent years, hair conditioning compositions which are not rinsed off from hair have become more and more popular.

Conditioners are used to improve combability. manageability, shine, elasticity, body and volume of hair. Despite the fact that the literature is reasonably well advanced, there are still areas to improve. Especially, in the area of conditioning compositions applied directly to dry hair has gained less attention, and therefore, further research in this particular area is still very much appreciated. Needless to mention, any conditioner which may be applied onto dry hair must be found very practical, especially by female users since there is no need of a prior cleansing step. In particular conditioners which do not result in volume loss after application are still to be provided, especially the ones applied directly onto dry hair.

Inventors of the present invention have surprisingly found out that a substantially non-aqueous composition comprising one or more alkyl methicone, one or more volatile organic solvent and one or more volatile isoalkane conditions hair excellently and improve hair shine, elasticity and manageability without affecting hair volume.

Accordingly, the first object of the present invention is a substantially non-aqueous composition for hair comprising one or more alkyl methicone, one or more volatile organic solvent and one or more volatile isoalkane with the condition that the composition comprises not more than 5% by weight, water, calculated to the total composition.

Further object of the present invention is the use of a composition comprising one or more alkyl methicone, one or more volatile organic solvent and one or more volatile isoalkane with the condition that the composition comprises not more than 5% by weight, water, calculated to the total composition, for conditioning hair, especially dry hair.

Another object of the present invention is a process for conditioning hair wherein a composition one or more alkyl methicone, one or more volatile organic solvent and one or more volatile isoalkane with the condition that the composition comprises not more than 5% by weight, water, calculated to the total composition is applied onto hair, especially dry hair.

Composition of the present invention is substantially non-aqueous and therefore comprises not more than 5% by weight, calculated to the total of the composition, water and preferably water content is utmost 2.5% and more preferably not more than 1% and most preferably compositions do not comprise any added water. It is worth to mention that the water content may especially result in because of the use of volatile organic solvent since they are as a rule hygroscopic and, therefore, comprises water.

The compositions of the present invention comprise one or more alkyl methicone at a concentration range of 0.01 to 15%, preferably 0.05 to 12.5% more preferably 0.1 to 10% and most preferably 0.1 to 7.5% by weight, calculated to total composition.

Non-limiting suitable examples are caprylyl methicone, cetearyl methicone, ethyl methicone, hexyl methicone, lauryl methicone, myristyl methicone and stearyl methicone and their mixtures. The most preferred is caprylyl methicone. Composition of the present invention comprises one or more volatile organic solvent, at a concentration of 20 to 70%, preferably 30 to 60%, more preferably, 40 to 50% by weight calculated to the total composition. Suitable and preferred ones are C₂ to C₄ straight or branched alcohols such as ethanol, propanol, isopropanol and n-butanol. Most preferred is ethanol.

Composition of the present invention comprises one or more volatile isoalkane at a concentration of 20 to 70%, preferably 30 to 60%, more preferably, 40 to 50% by weight calculated to the total of the composition. Suitable and the preferred one is isododecane, isohexadecane and isopraffin. The most preferred is isododecane

Compositions of the present invention comprise preferably one or more volatile silicone compound. Suitable volatile silicones are dimethicones and cyclomethicones and mixtures thereof.

Suitable volatile dimethicones are the ones with a viscosity of less than 5 mm²/s. The most preferred are dimethicone with a viscosity of 1 mm²/s nd 0.65 mm²/s which are available from Dow Corning with the trade name DC 200 Fluid.

Suitable volatile cyclomethicones are according to general formula 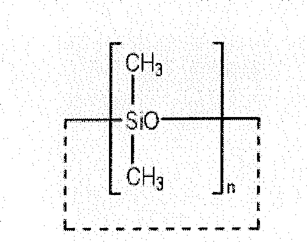 where n is a number between 3 and 7. Preferred are cyclopentasiloxanes known with the trade name for example Dow Corning 245.

Concentration of one or more volatile silicones in the composition is in the range of 0.01 to 15%, preferably 0.05 to 12.5% more preferably 0.1 to 10% and most preferably 0.1 to 7.5% by weight calculated to total composition.

It should be noted that within the content of the present invention alkyl methicones are not meant with the term volatile silicones.

In a preferred embodiment of the present invention, one or more organic solvent and one or more volatile isoalkanes are comprised at a weight ratio of 1.5:1 to 1:1.5 and most preferably 1:1.

Compositions of the present invention preferably comprise one or more UV filter, especially one or more oil soluble UV filter. Suitable ones are 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2,4-dihydroxybenzophenone, 2,2',4,4'-tetrahydroxybenzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4,4'-dimethoxy-5,5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher, 2-ethylhexyl-2-cyano-3,3-diphenyl acrylate also known as Octocrylene and/or ethylhexyl methoxycinnamate.

The most preferred are ethylhexyl methoxycinnamate and 2-hydroxy-4-methoxybenzophenone (Benzophenone-3).

The preferred concentration of the UV-absorber ranges from about 0.01 % to 2.5%, more preferably from 0.05 % to 1 % by weight, calculated to the total composition.

Compositions of the present invention comprise preferably one or more non-volatile oil at a concentration not higher than 2%, preferably not higher than 1.5%, more preferably not higher than 1% and most preferably not higher than 0.75% by weight calculated to the total of the composition.

Suitable ones are non-volatile silicones such as arylated silicones for example phenyl methicone, phenyl trimethicone, diphenyl dimethicone, diphenylsiloxy phenyl trimethicone, tetramethyl tetraphenyl trisiloxane, triphenyl trimethicone and trimethyl pentaphenyl trisiloxane known with the trade name Dow Corning 556, and dimethicones with higher viscosity, natural oils such as olive oil, almond oil, avocado oil, ricinus oil, coconut oil, palm oil, sesame oil, peanut oil, whale oil, sunflower oil, peach kernel oil, wheat germ oil, macadamia nut oil, night primrose oil, jojoba oil, castor oil, or soya oil, lanolin and the derivatives thereof and fatty acid alcohol esters according to the general structure

R₁ C(O) O R₂

wherein R₁ is an alkyl chain which may be straight or branched and saturated or unsaturated with 8 to 22 C atoms and R₂ is straight or branched alkyl chain with 2 to 4 C atoms such as isopropyl myristate, isopropyl laurate, isopropyl stearate, isopropyl palmitate, isopropyl oleate, isopropyl behenate, isopropyl linoleate, isopropyl isostearate, isopropyl arachidate, isobutyl myristate, isobutyl palmitate, and isobutyl stearate.

Most preferred non-volatile oils are fatty acid alcohol esters according to the above given general structure and among the suitable ones, with isopropyl myristate particularly exceptional conditioning properties are observed. Compositions of the present invention may furthermore comprise natural plant extracts especially of oil soluble ones such as Mentha piperita oil and Vitis vinifera seed oil.

Compositions of the present invention may furthermore comprise ingredients found customarily in cosmetic compositions such as fragrance, antioxidants, polyols, vitamins, amono acids and other well documented ones. It should be noted that when selecting any additional ingredient solubility and/or dispersability of such compounds in the remaining composition must be well examined in order to secure stability during the self life.

Compositions of the present invention may be applied in any form but spraying from a non pressurised container with a suitable spraying head is found to be the most convenient way of application in order to secure the optimal spreading on hair. Thus, anther object of the present invention is a product comprising a container wherein a composition according to present invention is comprised which has additionally a mounted or screwed spraying head.

Compositions of the present invention may conveniently be provided in a form of kit in addition to the other products. Thus, another object of the present invention is a kit for hair comprising one r more products wherein one of the products is a composition according to present invention.

Following example is to illustrate the invention but not to limit.

### Example 1

| | % by weight |
|---|---|
| Isododecane | 38.5 |
| Ethanol | 57.5 |
| Caprylyl methicone | 4.0 |

Above composition was prepared by combining all the liquid raw materials and mixing them well.

Above composition provides hair excellent light conditioning. Hair treated with the above composition does not loose its volume and receives enhanced body and shine.

Similar results are observed with the compositions below.

### Example 2

| | % by weight |
|---|---|
| Isododecane | 40.0 |
| Ethanol | 50.0 |
| Caprylyl methicone | 2.0 |
| Cyclopentasiloxane | 8.0 |

### Example 3

| | % by weight |
|---|---|
| Isododecane | 50.0 |
| Ethanol | 40.0 |
| Caprylyl methicone | 3.0 |
| Cyclopentasiloxane | 6.0 |
| Isopropyl palmitate | 1.0 |

### Example 4

| | % by weight |
|---|---|
| Isododecane | 43.0 |
| Ethanol | 43.0 |
| Caprylyl methicone | 5.0 |
| Dimethicone 1 cSt. | 8.0 |
| Isopropyl palmitate | 0.9 |
| Ethylhexyl methoxycinnamate | 0.1 |

## Claims

1. A substantially non-aqueous composition for hair **characterized in that** it comprises one or more alkyl methicone, one or more volatile organic solvent and one or more volatile isoalkane, with the condition that the composition comprises not more than 5% by weight, water, calculated to the total composition.

2. The composition according to claim 1 **characterized in that** alkyl methicone is selected from caprylyl methicone, cetearyl methicone, ethyl methicone, hexyl methicone, lauryl methicone, myristyl methicone and stearyl methicone and their mixtures.

3. Composition according to claims 1 and 2 **characterized in that** it comprises as an alkyl methicone caprylyl methicone and at a concentration in the range of 0.01 to 15% by weight calculated to total composition.

4. Composition according to any of the preceding claims **characterized in that** one or more volatile organic solvent is selected from C₂ to C₄ straight or branched alcohols.

5. Composition according to any of the preceding claims **characterized in that** it comprises ethanol as a volatile organic solvent and at a concentration in the range of 20 to 70% by weight calculated to the total composition.

6. Composition according to any of the preceding claims **characterized in that** one or more isoalkane is selected from isododecane, isohexadecane and isopraffin.

7. Composition according to any of the preceding claims **characterized in that** it comprises isododecane as and volatile isoalkane and at a concentration in the range of 20 to 70% by weight calculated to the total of the composition.

8. Composition according to any of the preceding claims **characterized in that** it comprises at least one or more additional volatile silicone compounds preferably selected from dimethicones and cyclomethicones.

9. Compositions according to any of the preceding claims **characterized in that** it comprise one or more additional volatile silicones at a concentration of 0.01 to 15% by weight calculated to the total of the composition.

10. Composition according to any of the preceding claims **characterized in that** it comprises one or more UV filter.

11. Composition according to any of the preceding claims **characterized in that** it comprises one or more non volatile oil.

12. Composition according to any of the preceding claims **characterized in that** it comprises fatty acid fatty alcohol ester according to general structure
R₁ C(O) O R₂
wherein R₁ is an alkyl chain which may be straight or branched and saturated or unsaturated with 8 to 22 C atoms and R₂ is straight or branched alkyl chain with 2 to 4 C atoms.

13. Use of composition according to any of the preceding claims for conditioning hair, especially dry hair.

14. Process for conditioning hair wherein a composition according to claims 1 to 12 is applied onto hair, especially dry hair.

15. Kit for hair comprising at least one composition according to claims 1 to 12.
